# EUROPEAN PATENT SPECIFICATION

(11) **EP 1 120 127 B1**
(45) Date of publication and mention of the grant of the patent: **11.08.2004**
(21) Application number: 00610012.7
(22) Date of filing: 28.01.2000
(51) Int. Cl.: A61M 25/00, A61M 25/01

(54) **Catheter made of a multifilar row of wires**
Katheter hergestellt aus einer Reihe von multifilamenten Drähten
Cathéter formé d'une rangée multibrins de fils

(43) Date of publication of application: 01.08.2001
(73) Proprietor: WILLIAM COOK EUROPE ApS, 4632 Bjaeverskov (DK); Cook Incorporated, Bloomington, IN 47402-0489 (US)
(72) Inventor: Klint, Henrik, Sonderskov, 2800 Lyngby (DK)
(74) Representative: Indahl, Peter Jensen

(56) References cited:
- US-A- 4 932 419
- US-A- 5 178 158
- US-A- 5 184 627
- US-A- 5 376 083
- US-A- 5 984 877

## Description

The present invention relates to a catheter having a distal end, a body portion and at least one lumen extending through the body portion in the longitudinal direction from a proximal end towards the distal end, the body portion is made of a multifilar helically wound row of wires provided with a sealing coating. Such a catheter is known from US-A-5 184 627 which discloses a pitch angle for the wires of about 15° to 18°.

Catheters for medical diagnostic or therapeutic use are well known. A wide variety of catheters exists for percutaneous insertion by the Seldinger technique into the vascular system to accomplish diagnostic or therapeutic objectives. The vessels of the peripheral vasculature have a relatively large diameter and low tortuosity, the coronary vasculature is somewhat smaller and more tortuous, and the vasculature in the soft tissue of the brain and liver is of small lumen and is very tortuous.

In order to be able to access the various parts of the vasculature the catheter needs to be flexible and to maintain its column strength when it follows a tortuous path. The contradictary requirements for flexibility and column strength are particularly pronounced in catheters for intercranial catheterizations used in a variety of diagnostic and interventional neurological techniques including delivery of contrast fluids, drugs or a vaso-occlusive agent, treatment of tumors, aneurysms, AVS (arterio-venous-shunts) etc.

It is an object of the present invention to provide a catheter with a delivery device which in its distal area is very flexible and yet easily pushable and capable of transferring torque.

In view of this, the catheter according to the invention is characterized in that the wires have a pitch angle in the range of 26°-76°.

By making the body portion of a row of two or more wires, which row is helically wound with a pitch roughly corresponding to the aggregate width of the adjacent wires in the row, the wound wires transfer torque and also force components directed in the axial direction of the catheter to the distal end thereof, and this construction is found to give a very high resistance to kinking of the catheter. When the catheter is heavily bent the cross-section of the catheter maintains a circular shape. This is an distinct advantage over prior art catheters which are deformed into oval shape when bent, and thus they are much more prone to kinking. The catheter surprisingly maintains its capabilities for transferring torque and push when it follows a tortuous path involving two or more loops, probably because of the excellent kinking resistance.

Due to the very high flexibility, pushability and torqueability and the ability of the construction to maintain each of these three characteristics even when set in a very tortuous pattern involving two or more tight loops, the catheter can be of use in very small and distant vessels such as deep brain sites accessed by intercranial catheterization.

If required, the flexibility of the distal portion of catheter during advancement along a tortuous path can be further increased by avoiding the use of a guidewire. The body portion of the catheter can be manoeuvred to the desired prosthesis deployment site like a guidewire because it is made of the multifilar helically wound row of wires so in terms of manoeuvrability there is no need for using the catheter in conjunction with a guidewire. However, a guidewire can be used to diminish the action of the catheter tip on the vascular wall because the tip will follow the guidewire when such is advanced in front of the catheter prior to pushing the catheter forward. It is an advantage of the catheter according to the present invention that the wall is primarily made of wires that provide a hard and relatively low-friction or slippery inner surface resulting in low resistance to advancing a member through the lumen of the catheter.

When the catheter is used without a guidewire in a soft tissue region it is preferred that the distal end of the catheter is provided with a buffer member, such as a soft obturator. The buffer member distributes the force from the catheter tip over a large area so that damage to the vascular wall is avoided.

In one embodiment the row of wires is made up of from 2 to 12 helically wound wires, preferably of from 4 to 8 helically wound wires. By using several wires their aggregate width can be adapted to correspond to the desired pitch for the given diameter of the catheter. A row of more than 12 wires has a tendency to buckle when the wires are helically wound in the common winding operation. For wires of round cross-sectional shape a number of from 4 to 8 wires in the row is preferred, but for flat wires or wires of oval shape two or three wires in a row can be more suitable.

In order to promote uniform and well-defined characteristics of the catheter along its length, the wires in the row can be located closely next to each other so that they mutually support each other. In this manner a possible deflection of a single wire strand is reduced to a minimum by the other wires in the row. As the wires in the row are wound into a helical course in a common movement there can be an interstice between the turns of the row of wires.

In an embodiment the wires in said row have a pitch angle in the range of 40°-65°. Although it is possible to use other pitch angles, angles chosen in these ranges provide a balanced solution to the requirements for the desired high flexibility, high column strength and fine torqueability. The range of 40°-65° is in particular useful for advancing the catheter to very distant, small sized vessels, such as in blood vessels in the brain, whereas the range of 35°-40° is applicable when very high flexibility is a dominant requirement, and the range of 70°-76° is applicable when very high pushability is a dominant requirement. It is of course possible to choose different pitch angles in different segments of the catheter.

At the time of performing the winding operation of the body portion, the individual wires in the row wound in the helical pattern have preferably a mainly circular cross-section. This facilitates the winding operation because twisting of a wire does not result in disorder in the row.

The sealing coating is preferably elastic. The wires are to a large extent mutually locked in position because several wires are wound in a common movement and thus one wire in the row is kept in place by the other wires in the row, but nevertheless some mutual movement can occur between the wires and in particular between the distal wire in one turn and the proximal wire in the consecutive turn. The sealing coating seals the interstices between the wires so that the catheter wall is leakproof. The elasticity of the sealing coating allows the wires to effect small mutual movements so that the excellent flexibility of the helically wound row of wires is maintained, and the elasticity also allows the catheter wall to stay leakproof when the wires move. The elasticity is a particular advantage when the catheter is pulled back as the pulling action can tend to elongate the body portion.

It is possible to provide the sealing coating only on the inner surface of the body portion which will result in a catheter of a very small wall thickness relative to its diameter. If a slightly enlarged diameter is acceptable the coating can also or as an alternative be placed on the outside of the body portion. The increase in diameter will be relatively modest as the sealing coating can be made thin. The sealing coating provided on the outside of the body portion can e.g. result in no more that a 5-15% increase of the outer diameter of the catheter body.

In an embodiment the sealing coating is a low-friction coating, such as a PTFE coating. A low-friction coating applied on the external side of the catheter wall acts to reduce the forces required to push forward the catheter inside a larger guiding catheter or a sheat, and a low-friction coating applied on the internal side of the catheter wall acts to reduce the forces required to push forward a guidewire or another means advanced through the catheter.

In yet an embodiment the sealing coating is a hydrophilic coating. Such a coating can traditionally be applied to the exterior of a catheter for reducing the tendency of the catheter to stick to the vascular wall, but according to the present invention in addition to the lubricating effect of the coating it also effects the sealing of the body portion.

The sealing coating is preferably thin and constitutes only a minor part of the wall thickness of the body portion. The thickness of the coating at the middle of the wire can be less than 0.1 mm, and preferably it is less than 0.02 mm.

It is possible to promote the flexibility of the catheter by machining the wires in said row to a lesser outer diameter, e.g. by grinding, at a region of the catheter. The region can extend along the whole length of the body portion, so that it is given a very precise outer dimension by the machining. In another embodiment the region is a distal region machined to a tapering shape with decreasing outer diameter in the distal direction causing the catheter to have an increasing flexibility towards the distal end which promotes introduction into very diminutive vessels. The reduced cross-sectional area of the wires produced by the machining greatly increases the bending flexibility of the catheter without sacrificing its ability to transfer torque.

It is preferred that the catheter at least in a 30 cm long distal area has a maximum outer diameter of less than 2.0 mm. A maximum diameter of 2 mm in the part of the catheter advanced through the vascular system allows for a straightforward percutaneous introduction by the Seldinger technique and easy navigation through the curves in the larger vessels. A maximum diameter of less than 1.00 mm allows introduction into quite fine and diminutive vessels such as into the external and internal carotid arteries. It is further possible to restrict the maximum outer diameter to at the most 0.75 mm which makes it possible to easily advance the catheter into e.g. the liver or other soft tissue areas, and by keeping the maximum outer diameter below 0.30 mm in a distal end area having a length of at least 10 cm even the most distant vascular regions are accessible and this embodiment of the catheter is excellent as a neuro-microcatheter.

In a further embodiment the number of wires in said helically wound row of wires varies along the length of the catheter. This can be attained by reducing, during the winding operation, the number of wires in the row. The lower number of wires in the row can be utilized to wind the wires with a larger pitch angle which increases the flexibility of the catheter. It is preferred that the number of wires diminishes in the distal direction so that the softness of the catheter increases without any change of material and without bonding together several separate catheter segments.

When the catheter has to traverse large lumen vascular paths in order to reach the more difficult small size vascular vessels, the helically wound row of wires can be stiffened in a proximal segment of said body portion by a supplementary tubular member, such as a cannula.

In the following, examples of the invention are descriped in more detail with reference to the very schematic drawings, on which
Fig. 1 is a side view of a catheter according to a preferred embodiment of the present invention,
Figs. 2 and 3 are enlarged partial views in longitudinal section of embodiments of the catheter in Fig. 1,
Fig. 4 is a partial view in longitudinal section of an embodiment where the number of wires in a row varies along the length of the catheter,
Fig. 5 is an enlarged partial and sectional view of the transition between two catheter segments having wires of different diameter,
Fig. 6 is an enlarged view of an embodiment having a catheter tip with a buffer member,
Fig. 7 depicts a winding operation on a multifilar row of wires,
Fig. 8 depicts a catheter segment having decreasing outer diameter, and
Fig. 9 is an illustration of the catheter in position in the vascular system.

In the following description of the depicted embodiments the same reference numerals are used for features of the same type.

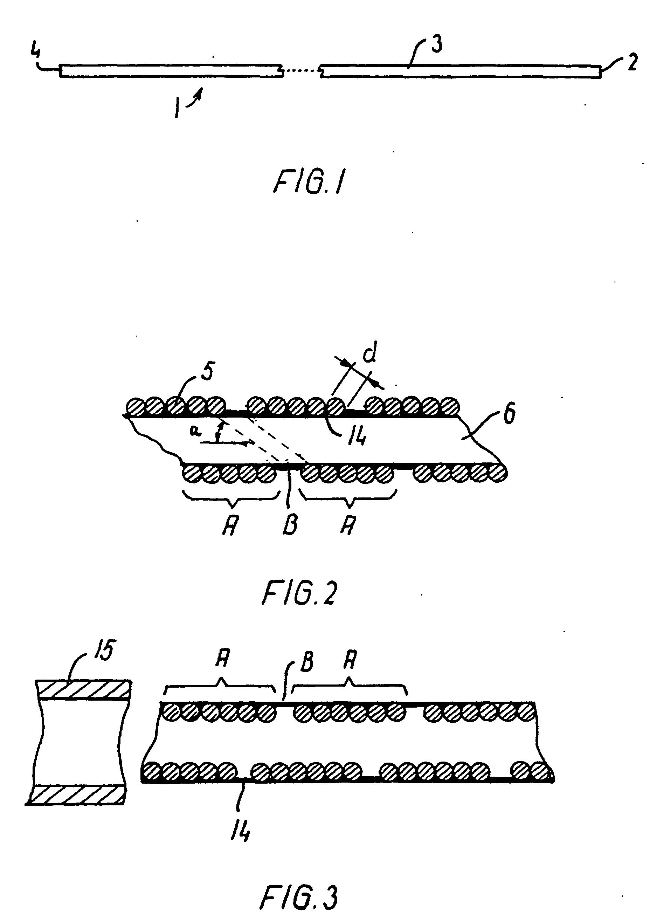

A catheter illustrated in Fig. 1 is generally denoted 1, and it has a distal end 2, a body portion 3 extending from the distal end to a proximal end 4. The body portion portion is made of a first helically wound multifilar row of wires 5 and it has a central longitudinally extending lumen 6. The catheter is normally open ended at both the proximal and the distal end, but for special uses, such as a single lumen balloon dilation catheter the distal end can be provided with means for barring the distal end opening.

For example, the catheter according to the present invention can be a balloon dilatation catheter used for percutaneous transluminal coronary angioplasty, an angiography catheter, a drug delivery catheter, a guiding catheter, an infusion catheter, etc.

The wires 5 used in the helically wound multifilar row are of a linear elastic material, such as stainless steel, titanium or tantalum, or it is made of a superelastic alloy, such as Nitinol. Preferably, the wires have an ultimate tensile strength in the range of 1800-2700 N/mm² but lower or higher values are also possible. The body portion 3 of the catheter is made by placing a group of from two to twelve wires in a row next to each other, e.g. according to the desired pitch angle, whereafter the group of wires is wound in a common movement into the body portion. Because a row of wires is wound, the individual wire is restricted in movement by the other wires and is plastically deformed into a permanent helical shape which is kept without any further restraints than the remaining wires in the row. The winding can be done on the inside end of a tubular support member where the row of wires are inserted at said end by rotating and simultaneously pushing the wires against the inside of the support. The wound wire then exits at the other end of the support. This produces a wire body with a very precise outer diameter.

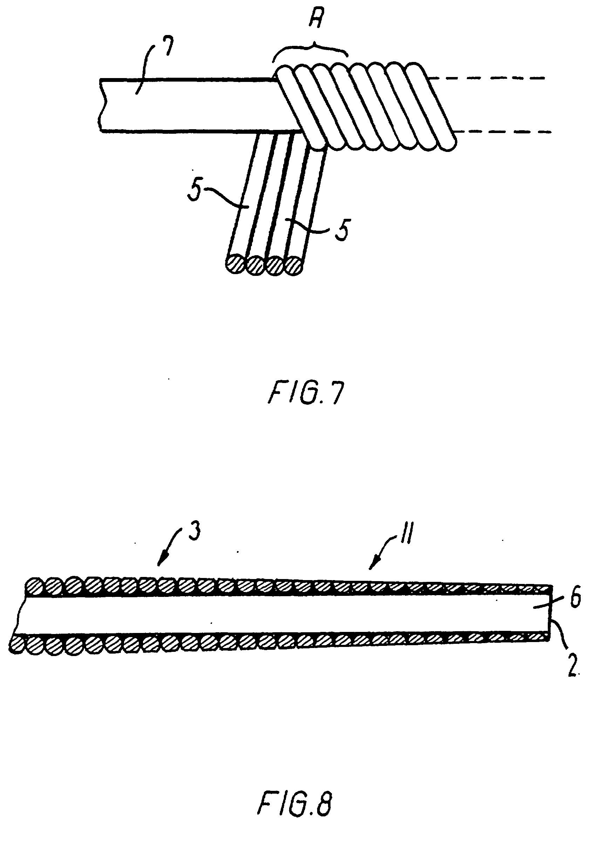

Alternatively, the winding operation can take place about a mandrel 7. Fig. 7 depicts winding of a row A of four identical wires 5. After the winding the mandrel with the coiled wires can be subjected to heat treatment in order to remove residual stresses from the wires. As an example the heat treatment can last for about two hours in an oven at a temperature of about 500°C. Generally, the temperature can be in the range of 400-600°C and the holding time at the temperature can last for many hours, such as up till 20 hours or more. After the heat treatment the mandrel is removed from the wires. The resulting helically wound multifilar row of wires maintain their mutual position even when heavily torqued, bent or pushed, presumably because each single wire is supported by the contiguous wires in the row. The winding operation can be effected so that the windings are touching each other, but preferably it is performed so that an interstice B is present between the turns (Fig. 2). The interstice facilitates bending of the body portion in thight turns.

The size of the pitch angle a depends on the diameter of the wires, the diameter of the body portion 3 and the number of wires in the row. The most preferred pitch angle a for the catheter is in the range of 40° - 65° or 50° - 70°. However, the combination of torque-transferral, pushability and transverse flexibility is normally well-balanced for pitch angles in the range of 50-68°. The diameter d of the wire is typically in the range of 0.06-0.75 mm, and preferably in the range of 0.15-0.45 mm.

In order to make the tip portion of the catheter more visible on a screen, it is desirable to use some kind of radiopaque material, such as platinum or gold. It can be of annular shape and be located at a predetermined distance from the distal end 2, or the terminal end of the distal tip of the catheter can be provided with a marker means for making it radiopaque, e.g. a gold layer or a gold thread.

The catheter can be made with uniform diameter throughout its length. In case the catheter has diminishing diameter towards the distal end a prefabricated catheter of uniform diameter can be grinded to the desired dimensions.

As an alternative or supplement to grinding the catheter can be composed of several segments in which the wires have mutually different diameters and cross-sectional areas. In a proximal segment 8 the wires can have a larger diameter than the wires in a distal segment 9. The segments can be joined together in axial extension by laser welding 10 as depicted in Fig. 5, by soldering, by bracing or in another manner such as mutual geometrically locking of the wires in the segments or by mechanical locking, such as press-fitting one segment into the lumen of the other segment, or binding the segments in axial extension with threads or suture.

When the catheter body is of multi-segment construction the inner lumens of the segments are preferably of even size which brings the advantage that an advancing guidewire can not grip onto a step in the inner wall of the body portion.

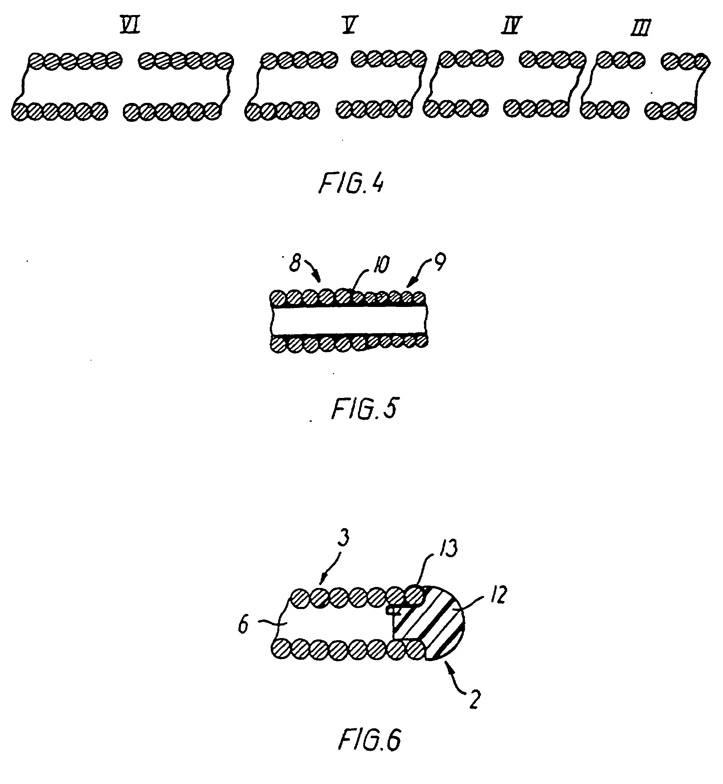

In the embodiment illustrated in Fig. 4 the number of wires in said helically wound row of wires varies along the length of the catheter. During the winding operation the number of wires in the row is reduced one by one at the points in time where the individual segment having a certain number of wires has obtained the desired length. The segment marked VI has six wires in the row, and the segments marked V, IV and III have five, four and three wires, respectively, in the row. Each time a wire is left out of the row, the pitch gets shorter and the pitch angle grows resulting in a even more flexible consecutive segment. The advantage of this embodiment is that the wires extending into the distal end segment are continuous from the distal end to the proximal end of the catheter, thus avoiding any need for joining the various segments. It is possible to secure the wire ends of the discontinous wires onto the other wires, such as by welding, soldering, etc.

A grinding procedure can also be used to produce a tapered segment 11 in the body portion portion 3. The taper can extend along a substantial length of the body portion. In the tapered segment the outer diameter of the catheter diminishes towards the distal end. Due to the taper the catheter obtains a gradually increasing transverse flexibility and a higher softness, but column stength and torque are nevertheless surprisingly transferred to the distal end.

When the catheter is to be advanced without a guidewire the distal end 2 can be provided with a soft buffer 12 having a rounded distal end which acts gently on the vascular wall when the catheter is pushed forward. A thread 14 can be securely embedded into the soft pliable material of buffer 12 and be ensnared around one of the distal wires, so that the thread will keep the buffer connected to the catheter body portion when the buffer is pushed out and cleared from the lumen of the catheter.

The wound wires 5 are provided with a sealing coating 14 on the inside or on the outside or on both sides of the catheter body. The coating is relatively thin and is preferably made of an elastic material which can be hydrophilic. The coating extends along the entire length of the catheter and is typically applied after winding and heat treatment of the catheter body have been completed. As an example the coating can be of PTFE applied onto the outside of the body portion in the same manner as such a coating is traditionally applied onto the exterior of a guidewire. When the coating is to be applied on the external and the internal sides of the body portion the catheter length can be dipped into a bath of liquid coating material which is then allowed to solidify.

The coating can comprise a hydrophilic polymer selected from the group comprising polyacrylate, copolymers comprising acrylic acid, polymethacrylate, polyacrylamide, poly(vinyl alcohol), poly(ethylene oxide), poly(ethylene imine), carboxymethylcellulose, methylcellulose, poly(acrylamide sulphonic acid), polyacrylonitril, poly(vinyl pyrrolidone), agar, dextran, dextrin, carrageenan, xanthan, and guar. The hydrophilic polymers can comprise ionizable groups such as acid groups, e.g. carboxylic, sulphonic or nitric groups. The hydrophilic polymers may be cross-linked through a suitable cross-binding compound. A cross-binder generally comprise two or more functional groups which provides for the connection of the hydrophilic polymer chains. The actual used cross-binder depends on the polymer system. If the polymer system is polymerized as a free radical polymerisation, a preferred cross-binder comprises 2 or 3 unsaturated double bonds.

In the follwing some examples of catheters made according to the invention are described.

### EXAMPLE 1:

A catheter was made of a helically wound row of 4 wires of 0.35 mm wire diameter d. The body portion of wound wires had initially an outside diameter of 1.67 mm and an inner lumen of 0.97 mm. A coating of PTFE of a minimum thickness of 0.1 mm was applied onto the inside of the catheter. The catheter was set in a complex curved shape involving three consecutive loops of a loop diameter of 24 mm axially separated by two loops of a loop diameter of 18 mm and a number of further turns representative of a complex vascular structure. Then the body portion of the catheter was manipulated and it proved to be easily pushed forward and retracted as well as easily torqued. Then a guidewire was pushed forward in relation to the body portion, and it proved to be easily pushed out past the distal end of the catheter without causing noticeable flexion or movement of the catheter.

### EXAMPLE 2:

A catheter was made of a helically wound row of 5 wires of 0.30 mm wire diameter d. The winding of a first segment of the body portion was made with an outside diameter of 1.20 mm and an inner lumen of 0.6 mm. Another segment was made up of a second helically wound row of four wires of 0.15 mm wire diameter. This segment had a length of 20 cm and an outside diameter of 1.20 mm and an inside diameter of 0.9 mm. The segments were jointed by laser welding. The catheter body was provided with a flexibel coating on its outside. The catheter was advanced through a complex curved vascular system involving several consecutive, retrograde turns in vessels having a lumen of only 2 mm and less. Then the torqued and moved both forwards and backwards without any problems.

### EXAMPLE 3:

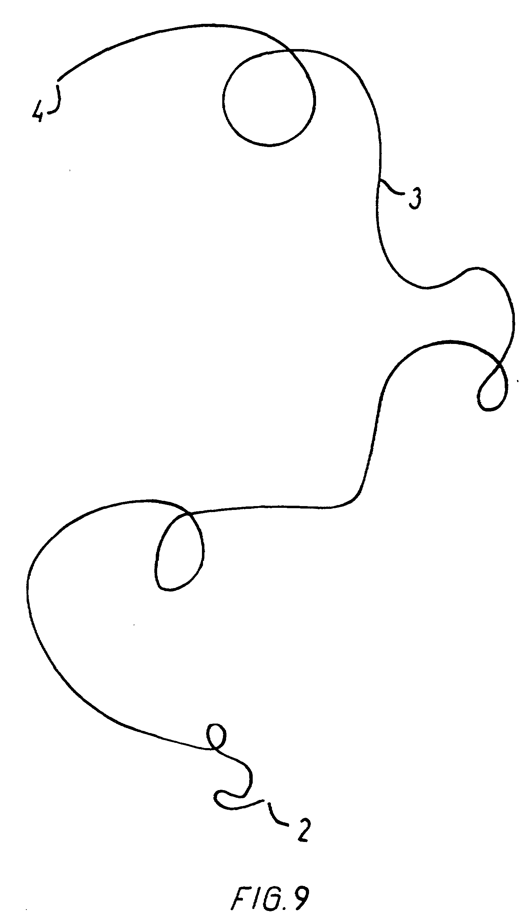

A catheter was made of a first helically wound row of 8 wires of 0.075 mm wire diameter d. The winding was made with an outside diameter of 0.25 mm and an inner lumen of 0.1 mm. The body portion had a length of 160 cm and was coated with a hydrophilic material of polyacrylamide on its outside. When tested the catheter showed no problems. After placing the catheter in a very complex pattern involving several sharp turns (see an example in Fig. 9) a guidewire could be advanced with only very low friction, and after removal of the guidewire, a fluid could be injected through the catheter without leakage through the coating.

When the catheter is to be introduced into the vascular system there is firstly established a percutaneous puncture site, e.g. by the Seldinger technique or an existing puncture site is used. Then the body portion of the catheter is inserted through the cannula, sheat or hemostatic valve at the puncture site and the catheter is advanced and navigated through the vascular system to the prosthesis deployment site. Due to the very high flexibility, pushability and torqueability of the catheter it can be advanced to the prosthesis deployment site without use of a guidewire, or a sheat to negotiate the sharp curves in the path. When large lumen vessels are to be traversed in order to enter the vasculature near the target site, it can be an advantage to stiffen the proximal portion of the catheter by inserting it through a cannula 14 ( Fig. 3) or another kind of a more rigid structure.

The catheter according to the invention can be used as a traditional catheter, and it can also be used as a sheath which has normally a shorter length than a traditional catheter.

Individual features of the various embodiments can be combined into further embodiments according to the present invention. It is possible to effect the sealing coating as a multilayer coating, e. g. comprising a primer-coating and a top-coat where the primer-coating is chosen to provide a strong bonding to the wires, and the top-coat provides the sealing action and can be a hydrophilic slippery coating providing a low friction surface.

## Claims

1. A catheter (1) having a distal end (2), a body portion (3) and at least one lumen (6) extending through the body portion (3) in the longitudinal direction from a proximal end (4) towards the distal end (2), the body portion (3) is made of a multifilar helically wound row of wires (5) provided with a sealing coating (14), **characterized in that** the wires have a pitch angle (α) in the range of 26°-76°.

2. A catheter according to claim 1, **characterized in that** the row wires (5) is made up of from 2 to 12 helically wound wires, preferably of from 4 to 8 helically wound wires.

3. A catheter according to claim 2, **characterized in that** the wires in the row (5) are located closely next to each other.

4. A catheter according to any one of claims 1-3, **characterized in that** the wires (5) have a pitch angle (α) in the range of 40°-65°.

5. A catheter according to any one of claims 1-4, **characterized in that** the body portion (3) is wound of wires (5) having a mainly circular cross-section.

6. A catheter according to any one of claims 1-5, **characterized in that** said sealing coating (14) is elastic.

7. A catheter according to any one of claims 1-6, **characterized in that** the sealing coating (14) is provided only on the inner surface of the body portion (3).

8. A catheter according to claims 7, **characterized in that** the sealing coating (14) is a low-friction coating, such as a PTFE coating.

9. A catheter according to any one of claims 1-8, **characterized in that** the coating (14) is a hydrophilic coating.

10. A catheter according to claim 9, **characterized in that** the thickness of the coating (14) at the middle of the wire is less than 0.1 mm, preferably less than 0.02 mm.

11. A catheter according to any one of the claims 1-10, **characterized in that** the wires (5) in said row are machined to a lesser outer diameter, e.g. by grinding, in a region (9) of the catheter.

12. A catheter according to claim 11, **characterized in that** said region is a distal region (9) machined to a tapering shape (11) with decreasing outer diameter in the distal direction.

13. A catheter according to any one of the preceding claims, **characterized in that** the catheter (1) at least in a 30 cm long distal segment (9) has a maximum outer diameter of less than 2.0 mm.

14. A catheter according to any one of the claims 1-13, **characterized in that** the catheter (1) is a micro-catheter with a 30 cm long distal segment (9) having a maximum outer diameter of less than 1.00 mm, preferably at the most 0.75 mm.

15. A catheter according to any one of the claims 1-14, **characterized in that** the catheter (1) is a neuro-microcatheter having a distal segment of a length of at least 10 cm which has a maximum outer diameter of less that 0.30 mm.

16. A catheter according to any one of the claims 1-15, **characterized in that** the number of wires (5) varies along the length of the catheter, preferably so that the number of wires diminishes in the distal direction.

17. A catheter according to claim 15 or 16, **characterized in that** in a proximal segment (8) the row of wires (5) is stiffened by a supplementary tubular member.

18. A catheter according to any one of the preceding claims, **characterized in that** the distal end (9) of the catheter (1) is provided with a buffer member (12), such as a soft obturator.

19. A catheter according to any one of the preceding claims, **characterized in that** the wires extending into the distal end segment (9) are continuous from the distal end to the proximal end of the catheter (1).

20. A catheter according to any one of the claims 1-14, **characterized in that** the catheter (1) is a neuro-microcatheter.

21. A catheter according to any one of the preceding claims, **characterized in that** the catheter (1) is open ended at both the proximal end (4) and the distal end (2).

## Patentansprüche

1. Katheter ( 1 ) mit einem Distalende ( 2 ), mit einem Körperteil ( 3 ) aus einer spiralförmig gewundenen Reihe von multifilamenten, mit einer Dichtungsschicht ( 14) versehenen Drähten (5) und mit zumindest einem Lumen ( 6 ), das sich durch das Körperteil ( 3 ) in Längsrichtung von einem nächstgelegenen Ende ( 4 ) zum Distalende ( 2 ) erstreckt, **dadurch gekennzeichnet, dass** die Drähte einen Neigungswinkel ( a ) im Bereich von 26° bis 76° aufweisen.

2. Katheter nach Anspruch 1, **dadurch gekennzeichnet, dass** die Reihe Drähte ( 5 ) aus 2 bis 12 spiralförmig gewickelten Drähten, bevorzugt aus 4 bis 8 spiralförmig gewickelten Drähten, aufweist.

3. Katheter nach Anspruch 2, **dadurch gekennzeichnet, dass** die Drähte in der Reihe ( 5 ) nahe bei einander liegen.

4. Katheter nach einem der Ansprüche 1 bis 3, **dadurch gekennzeichnet, dass** die Drähte einen Neigungswinkel ( a ) im Bereich von 40° bis 65° aufweisen.

5. Katheter nach einem der Ansprüche 1 bis 4, **dadurch gekennzeichnet, dass** das Körperteil ( 3 ) aus Drähten ( 5 ) gewunden ist, die hauptsächlich einen kreisförmigen Querschnitt aufweisen.

6. Katheter nach einem der Ansprüche 1 bis 5, **dadurch gekennzeichnet, dass** die Dichtungsschicht ( 14 ) elastisch ist.

7. Katheter nach einem der Ansprüche 1 bis 6, **dadurch gekennzeichnet, dass** die Dichtungsschicht ( 14 ) nur auf der inneren Oberfläche des Körperteils ( 3 ) aufgebracht wird.

8. Katheter nach einem der Ansprüche 1 bis 7, **dadurch gekennzeichnet, dass** die Dichtungsschicht ( 14 ) eine Beschichtung mit geringer Reibung wie etwa eine PTFE Schicht ist.

9. Katheter nach einem der Ansprüche 1 bis 8, **dadurch gekennzeichnet, dass** die Schicht ( 14 ) eine hydrophile Beschichtung ist.

10. Katheter nach Anspruch 9, **dadurch gekennzeichnet, dass** die Dicke der Schicht ( 14 ) in der Mitte des Drahtes weniger als 0,1 mm, bevorzugt weniger als 0,02 mm beträgt.

11. Katheter nach einem der Ansprüche 1 bis 10, **dadurch gekennzeichnet, dass** die Drähte in der Reihe in einem Bereich des Katheters zum Beispiel durch Schleifen maschinell auf einen geringeren äußeren Durchmesser gebracht werden.

12. Katheter nach Ansprüche 11, **dadurch gekennzeichnet, dass** der Bereich ein Distalbereich ( 9 ) ist, der maschinell in eine konische Form mit abnehmendem äußeren Durchmesser in Distalrichtung gebracht wird.

13. Katheter nach einem der vorangehenden Ansprüche, **dadurch gekennzeichnet, dass** der Katheter ( 1 ) in einem zumindest 30 cm langen Distalsegment ( 9 ) einen maximalen äußeren Durchmesser von weniger als 2,0 mm aufweist.

14. Katheter nach einem der vorangehenden Ansprüche 1 bis 13, **dadurch gekennzeichnet, dass** der Katheter ( 1 ) ein Mikrokatheter mit einem 30 cm langen Distalsegment ( 9 ) mit einem maximalen äußeren Durchmesser von weniger als 1,00 mm, bevorzugt maximal 0,75 mm, ist.

15. Katheter nach einem der Ansprüche 1 bis 14, **dadurch gekennzeichnet, dass** der Katheter ( 1 ) ein Neuro - Mikrokatheter ist, der ein Distalsegment von zumindest 10 cm Länge beinhaltet, das einen maximalen äußeren Durchmesser von weniger als 0,30 mm aufweist.

16. Katheter nach einem der Ansprüche 1 bis 15, **dadurch gekennzeichnet, dass** die Anzahl der Drähte ( 5 ) entlang der Länge des Katheters variiert, bevorzugt so, dass sich die Anzahl der Drähte in Distalrichtung verringert.

17. Katheter nach Anspruch 15 oder 16, **dadurch gekennzeichnet, dass** in einem nächstgelegenen Segment ( 8 ) die Reihe von Drähten ( 5 ) von einem röhrenförmigen Zusatzglied verstärkt wird.

18. Katheter nach einem der vorangehenden Ansprüche, **dadurch gekennzeichnet, dass** das Distalende ( 9 ) des Katheters ( 1 ) ein Pufferglied ( 12 ), wie etwa ein weiches Obturat aufweist.

19. Katheter nach einem der vorangehenden Ansprüche, **dadurch gekennzeichnet, dass** die sich in das Distalendsegment ( 9 ) erstreckenden Drähte vom Distalende bis zum nächstgelegenen Ende des Katheter ( 1 ) fortlaufend sind.

20. Katheter nach einem der Ansprüche 1 bis 14, **dadurch gekennzeichnet, dass** der Katheter ( 1 ) ein Neuro - Mikrokatheter ist.

21. Katheter nach einem der vorangehenden Ansprüche, **dadurch gekennzeichnet, dass** der Katheter ( 1 ) an sowohl dem nächstgelegenen Ende ( 4 ) und dem Distalende ( 2 ) ein offenes Ende aufweist.

## Revendications

1. Cathéter (1) ayant une extrémité distale (2), une portion de corps (3) et au moins une lumière (6) s'étendant à travers la portion de corps (3) dans la direction longitudinale depuis une extrémité proximale (4) vers l'extrémité distale (2), la portion de corps (3) est faite d'une ligne de fils multibrins enroulés de manière hélicoïdale (5) munie d'un revêtement scellant (14), **caractérisé en ce que** les fils ont un angle d'hélice (α) dans la plage de 26° à 76 °.

2. Cathéter selon la revendication 1, **caractérisé en ce que** la ligne de fils (5) est constituée de 2 à 12 fils enroulés de manière hélicoïdale, de préférence de 4 à 8 fils enroulés de manière hélicoïdale.

3. Cathéter selon la revendication 2, **caractérisé en ce que** les fils dans la ligne (5) sont situés à proximité rapprochée les uns des autres.

4. Cathéter selon l'une quelconque des revendications 1 à 3, **caractérisé en ce que** les fils (5) ont un angle d'hélice (α) dans la plage de 40 ° à 65 °.

5. Cathéter selon l'une quelconque des revendications 1 à 4, **caractérisé en ce que** la portion de corps (3) est entourée de fils (5) ayant une section en coupe essentiellement circulaire.

6. Cathéter selon l'une quelconque des revendications 1 à 5, **caractérisé en ce que** ledit revêtement scellant (14) est élastique.

7. Cathéter selon l'une quelconque des revendications 1 à 6, **caractérisé en ce que** le revêtement scellant (14) est fourni uniquement sur la surface interne de la portion de corps (3).

8. Cathéter selon la revendication 7, **caractérisé en ce que** le revêtement scellant (14) est un revêtement à coefficient de frottement réduit, tel qu'un revêtement en PTFE.

9. Cathéter selon l'une quelconque des revendications 1 à 8, **caractérisé en ce que** le revêtement (14) est un revêtement hydrophile.

10. Cathéter selon la revendication 9, **caractérisé en ce que** l'épaisseur du revêtement (14) au milieu de fil est inférieure à 0,1 mm, de préférence inférieure à 0,02 mm.

11. Cathéter selon l'une quelconque des revendications 1 à 10, **caractérisé en ce que** les fils (5) dans ladite ligne sont usinés avec un diamètre externe inférieur, par exemple par meulage, dans une région (9) du cathéter.

12. Cathéter selon la revendication 11, **caractérisé en ce que** ladite région est une région distale (9) usinée en une forme conique (11) avec un diamètre externe décroissant dans la direction distale.

13. Cathéter selon l'une quelconque des revendications précédentes, **caractérisé en ce que** le cathéter (1) a, au moins dans un segment distal de 30 cm de long (9) un diamètre externe maximum inférieur à 2,0 mm.

14. Cathéter selon l'une quelconque des revendications 1 à 13, **caractérisé en ce que** le cathéter (1) est un micro-cathéter avec un segment distal de 30 cm de long dont un diamètre externe maximum est inférieur à 1,00 mm, de préférence au plus 0,75 mm.

15. Cathéter selon l'une quelconque des revendications 1 à 14, **caractérisé en ce que** le cathéter (1) est un neuro-microcathéter ayant un segment distal d'une longueur d'au moins 10 cm qui a un diamètre externe maximum inférieur à 0,30 mm.

16. Cathéter selon l'une quelconque des revendications 1 à 15, **caractérisé en ce que** le nombre de fils (5) varie le long de la longueur du cathéter, de préférence de manière à ce que le nombre de fils diminue dans la direction distale.

17. Cathéter selon la revendication 15 ou 16, **caractérisé en ce que** dans un segment proximal (8) la ligne de fils (5) est raidie par un élément tubulaire supplémentaire.

18. Cathéter selon l'une quelconque des revendications précédentes, **caractérisé en ce que** l'extrémité distale (2) du cathéter (1) est munie d'un élément tampon (12) tel qu'un obturateur souple.

19. Cathéter selon l'une quelconque des revendications précédentes, **caractérisé en ce que** le fils s'étendant dans le segment d'extrémité distale (2) sont continus depuis l'extrémité distale jusqu'à l'extrémité proximale du cathéter (1).

20. Cathéter selon l'une quelconque des revendications 1 à 14, **caractérisé en ce que** le cathéter (1) est un neuro-microcathéter.

21. Cathéter selon l'une quelconque des revendications précédentes, **caractérisé en ce que** le cathéter (1) a des extrémités ouvertes au niveau de l'extrémité proximale (4) et de l'extrémité distale (2).
